# DEMANDE DE BREVET EUROPEEN

(11) **EP 2 305 206 A1**
(43) Date de publication de la demande: **06.04.2011**
(21) Numéro de dépôt: 10178618.4
(22) Date de dépôt: 23.09.2010
(51) Int. Cl.: A61K 8/06, A61K 8/34, A61K 8/368, A61K 8/39, A61K 8/49, A61K 8/97, A61Q 19/06, A61K 36/185, A61K 36/82, A61K 36/74, A61K 36/77

(54) **Composition amincissante**

(30) Priorité: 29.09.2009 FR 0956723
(71) Demandeur: L'Oréal, 75008 Paris (FR)
(72) Inventeur: Pierre, Patricia, 92160, Antony (FR); Fonolla Moreno, Angeles, 75013, Paris (FR)
(74) Mandataire: Kromer, Christophe

(57) **Abrégé**

L'invention concerne une composition comprenant, dans un milieu physiologiquement acceptable, au moins une base xanthique ou un extrait végétal en contenant, et au moins un composé oxyalkyléné particulier.

L'invention concerne également un procédé cosmétique pour lutter contre la cellulite et/ou la peau d'orange et/ou amincir la silhouette, comprenant l'application sur la peau de la composition.

La composition appliquée sur la peau présente de bonnes propriétés cosmétiques de douceur et de non collant.

## Description

La présente invention a pour objet une composition cosmétique comprenant une base xanthique et un composé oxyalkyléné particulier. L'invention a également pour objet un procédé cosmétique pour lutter contre la cellulite et/ou la peau d'orange et/ou amincir la silhouette, comprenant l'application topique de la composition sur la peau.

L'adiposité (ou excès de graisse dans le tissu cellulaire sous-cutané) peut avoir de nombreuses causes plus ou moins complexes.

Certaines cellules de la peau, appelées adipocytes, contiennent des quantités variables de graisses sous la forme de triglycérides, ces triglycérides étant synthétisés *in vivo* par les adipocytes eux-mêmes, selon des réactions de type enzymatique (lipogénèse), à partir des acides gras libres et du glucose (après dégradation de ce dernier en glycérol) contenus dans l'organisme et apportés à celui-ci par l'intermédiaire de certains aliments. Or, parallèlement, les triglycérides ainsi formés, puis stockés, dans les cellules adipocytaires peuvent également se redécomposer, toujours sous l'action d'enzymes spécifiques (lipolyse) contenues dans ces mêmes cellules, en libérant cette fois des acides gras d'une part et du glycérol et/ou des mono- et/ou des di-esters du glycérol d'autre part. Les acides gras ainsi relargués peuvent alors soit diffuser dans l'organisme pour y être consommés ou transformés de différentes façons, soit être recaptés (aussitôt ou un peu plus tard) par les adipocytes pour générer à nouveau des triglycérides par lipogénèse.

Si, pour des raisons diverses (nourriture trop riche, inactivité, vieillissement et autres), un déséquilibre substantiel s'installe dans l'organisme entre la lipogénèse et la lipolyse, c'est à dire plus précisément si les quantités de graisses formées par lipogénèse deviennent notablement et constamment supérieures à celles qui sont éliminées par lipolyse, il se produit alors dans les adipocytes une accumulation de triglycérides qui, si elle devient excessive, peut se traduire progressivement par l'apparition d'une peau épaisse, à surface souvent irrégulière ("peau d'orange") et de consistance plus ou moins flasque ou gélatineuse, donnant finalement à la silhouette un aspect général disgracieux pouvant évoluer entre la simple surcharge locale (lipodismorphie) et la formation de cellulite, en passant par l'embonpoint certain, et enfin la réelle obésité.

Or, compte tenu notamment du profond inconfort tant physique qu'esthétique, et parfois psychologique, qu'elles occasionnent auprès des individus qui en sont atteints, en particulier chez les femmes, l'adiposité et la cellulite constituent de nos jours une affection de moins en moins bien supportée ou acceptée.

Des solutions ont donc été proposées dans l'art antérieur pour intervenir sur le métabolisme des acides gras qui est, comme on l'a vu, l'une des cibles privilégiées dans le contrôle de cette surcharge lipidique adipocytaire.

Celui-ci peut être modulé :
- soit par blocage du transport du glucose à l'intérieur de l'adipocyte qui conduit, comme on l'a vu, à une diminution des acides gras entrant dans l'adipocyte,
- soit par inhibition de la lipoprotéine lipase,
- soit par activation de la triglycéride lipase (ou lipase hormonosensible), généralement en stimulant l'AMP cyclique, par exemple par activation de l'adényl cyclase, ou en provoquant son accumulation par inhibition de la phosphodiestérase.

D'autres voies biologiques ont été explorées pour agir sur le mécanisme de la lipogénèse et/ou de la lipolyse. Il a ainsi été proposé d'utiliser des antagonistes des récepteurs du neuropeptide Y (NPY), qui est un neuromédiateur intervenant dans un certain nombre de processus physiologiques et dont l'implication dans la régularisation de la lipolyse a pu être démontrée (P. Valet, J. Clin. Invest., 1990, 85, 291-295). On peut également utiliser des antagonistes des récepteurs α_{z} ou encore des agonistes des récepteurs β3-adrénergiques.

Les compositions cosmétiques proposées jusqu'à présent en vue de traiter l'adiposité contiennent donc des composés dits amincissants qui agissent sur un ou plusieurs des mécanismes mentionnés précédemment. Parmi ceux-ci, on peut plus particulièrement citer les bases xanthiques (i.e. des dérivés de la xanthine), telles que la théophylline, la caféine, la théobromine.

Toutefois, les bases xanthiques comme la caféine ont l'inconvénient de conférer aux compositions amincissantes de mauvaises propriétés cosmétiques ressenties par l'utilisatrice après l'application de la composition sur la peau. La peau traitée présente un effet collant, une sensation de tiraillement, un manque de douceur: ces propriétés néfastes ne satisfont pas l'utilisatrice.

Le but de la présente invention est donc de disposer d'une composition cosmétique à action amincissante contenant une base xanthique et présentant de bonnes propriétés cosmétiques après l'application de la composition sur la peau, notamment de bonnes propriétés de non collant et de douceur.

Les inventeurs ont découvert qu'une telle composition peut être obtenue en utilisant avec la base xanthique un dérivé oxyalkyléné de formule (I) tel que décrit ci-après.

La composition appliquée sur la peau ne présente pas d'effet collant et confère une sensation de douceur agréable sur la peau, sans effet de tiraillement. En outre, la composition présente une bonne efficacité amincissante.

De façon plus précise, l'invention a pour objet une composition comprenant, dans un milieu aqueux physiologiquement acceptable, au moins une base xanthique ou un extrait végétal en contenant, au moins un dérivé oxyalkyléné de formule (I) suivante:

Z-{O(AO)ₗ(EO)ₘ-(BO)ₙH}ₐ (I)

dans laquelle
Z représente un radical obtenu par élimination d'un ou plusieurs groupes hydroxyle d'un composé comprenant de 3 à 9 groupes hydroxyle ;
AO représente une groupe oxyalkyléné comprenant de 3 à 4 atomes de carbone
EO représente un groupe oxyéthylène;
BO représente un groupe oxyalkylène comprenant 4 atomes de carbone, avade3à9;
I, m, et n représentent respectivement le nombre moyen de moles d'unités AO, EO and BO, et 1 ≤ l ≤ 50, 1 ≤ m ≤ 50 et 0.5 ≤ n ≤ 5;
le ratio en poids de AO sur EO (AO/EO) allant de 1/5 à 5/1.

L'invention a également pour objet un procédé cosmétique pour lutter contre la cellulite et/ou la peau d'orange et/ou amincir la silhouette, comprenant l'application sur la peau d'une composition telle que définie précédemment.

La composition selon l'invention est généralement adaptée à une application topique sur la peau et comprend donc généralement un milieu physiologiquement acceptable, c'est-à-dire compatible avec la peau. Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.

La composition selon l'invention comprend au moins une base xanthique ou un extrait végétal en contenant.

Parmi les bases xanthiques utilisables selon l'invention, on peut citer : la caféine, la théophylline, la théobromine, l'acéfylline, le nicotinate de xanthinol, la diniprophylline, la diprophylline, l'étamiphylline et ses dérivés, l'étophylline, la proxyphylline, la pentophylline, la propentophylline, la pyridophylline et la bamiphylline, sans que cette liste ne soit limitative.

On préfère en particulier utiliser la caféine, la théophylline, la théobromine et l'acéfylline. On utilise de préférence la caféine. Ces bases xanthiques sont connues comme inhibiteurs de phosphodiestérase, qui est l'enzyme responsable de la dégradation de l'AMPc. En augmentant le taux intra-cellulaire d'AMPc, ces bases xanthiques favorisent l'activité lipolytique et constituent donc des actifs amincissants de premier ordre.

Comme exemples d'extraits végétaux renfermant des bases xanthiques, on peut notamment citer les extraits de thé, de café, de guarana, de maté et de cola, sans que cette liste ne soit limitative.

La base xanthique peut être présente dans la composition selon l'invention en une teneur allant de 0,5 % à 10 % en poids, par rapport au poids total de la composition, de préférence allant de 1 % à 7 % en poids, et préférentiellement allant de 2 % à 7 % en poids.

Le dérivé oxyalkyléné utilisé dans la présente invention répond à la formule (I) suivante :

Z-[O(AO)ₗ(EO)ₘ-(BO)ₙH}ₐ (I)

dans laquelle
Z représente un radical obtenu par élimination d'un ou plusieurs groupes hydroxyle d'un composé comprenant de 3 à 9 groupes hydroxyle ;
AO représente une groupe oxyalkyléné comprenant de 3 à 4 atomes de carbone EO représente un groupe oxyéthylène;
BO représente un groupe oxyalkylène comprenant 4 atomes de carbone, a va de 3 à 9;
I, m, and n représentent respectivement le nombre moyen de moles d'unités AO, EO et BO, et < ≤ l ≤ 50, 1 ≤ m ≤ 50 et 0.5 ≤ n ≤ 5;
le ratio en poids de AO sur EO (AO/EO) allant de 1/5 à 5/1.

Dans la composition selon l'invention, on peut utiliser un dérivé oxyalkyléné ou un mélange de dérivés oxyalkylénés identiques ou différents.

Dans le dérivé oxyalkyléné de formule (I), Z représente un radical obtenu par élimination d'un ou plusieurs groupes hydroxyle d'un composé comprenant de 3 à 9 groupes hydroxyle, et a représente le nombre de groupe hydroxyle du dérivé et va de 3 à 9.

Comme composés comprenant de 3 à 9 groupes hydroxyle, on peut citer par exemple, dans le cas où a = 3 : la glycérine, le trimethylolpropane ; dans le cas où a = 4 : l'erythritol, le pentaerythritol, le sorbitol, les alkylglycosides, la diglycérine ; dans le cas où a = 5 : le xylitol ; dans le cas où a = 6 : le dipentaerythritol, le sorbitol, l'inositol; dans le cas où a = 8, le sucrose, le trehalose; dans le cas où a = 9, le maltitol ; ou un mélanges desdits composés.

De préférence, Z représente un radical obtenu par élimination de groupe(s) hydroxyle d'un composé comprenant de 3 à 9 groupes hydroxyle, et 3 ≤ a ≤ 6. Comme composés préférés comprenant de 3 à 9 groupes hydroxyle, on peut citer la glycérine ou le trimethylolpropane, et en particulier la glycérine.

Lorsque a est inférieur ou égal à 2, la compatibilité entre le composé oxyalkyléné et la phase grasse de la composition tels que les huiles et corps gras est faible, et la stabilité du mélange dans des formulations à base d'huiles est affectée. Lorsque a est supérieur ou égal à 10, un effet collant est observé.

AO représente un groupe oxyalkyléné comprenant de 3 à 4 atomes de carbone. AO peut par exemple représenter un groupe oxypropylène, oxybutylène (oxy-n-butylène , oxyisobutylène ou oxy-t-butylène), un groupe oxytriméthylène, un groupe oxytetramethylène ou un mélange. AO est de préference choisi parmi les groupes oxypropylène et/ou oxybutylène, et de préférence encore parmi les groupes oxypropylène.

I représente le nombre moyen de moles d'unités AO et 1 ≤ l ≤ 50, de préférence 2 ≤ l ≤ 20.

m représente le nombre moyen de moles d'unités EO, et 1 ≤ m ≤ 50, de préférence 2 ≤ m ≤ 20. Lorsque I est égal à 0, un effet collant est constaté ; d'autre part, lorsque I dépasse 50, l'effet hydratant du composé (I) est réduit.

En outre, lorsque m est égal à 0, l'effet hydratant 50 décroît et lorsque m dépasse 50, un effet collant est constaté.

Le ratio en poids de AO sur EO (AO/EO) va de 1 /5 à 5/1, et de préférence 1/4 à 4/1. Lorsque ce ratio est inférieur à 1/5, un effet collant est constaté, et lorsque le ratio AO/EO dépasse 5/1, l'effet hydratant diminue.

L'ordre d'addition des unités AO et EO n'est pas décisif, les unités AO et EO peuvent être additionnées de manière statistique ou sous forme séquencée (blocs). Pour obtenir un effet améliorée de prévention de la sécheresse cutanée, AO et EO sont de préférence ajoutés de manière statistique.

BO représente un groupe oxyalkylène comprenant 4 atomes de carbone, et peut être par exemple choisi parmi les groupes oxybutyléne (tels que les groupes oxy-n-butylène, oxy-isobutylène ou oxy-t-butylène), oxytetramethylène et leurs mélanges. De préférence BO est choisi parmi les groupes oxybutylène.

n représente le nombre moyen de moles d'unités BO, et 0,5 ≤ n ≤ 5, de préférence 0,8 ≤ n ≤ 3, et mieux 1 ≤ n ≤ 3.

Lorsque n est inférieur à 0,5 on observe un effet collant. Lorsque n dépasse 5, l'effet hydratant diminue.

Dans la formule (I), les unités (BO)ₙ sont nécessairement liées à l'atome d'hydrogène terminal du dérivé oxyalkyléné.

Les dérivés oxyalkylénés de formule (I) peuvent être préparés par des méthodes bien connues, comme par exemple par polymérisation d'addition d'oxyde d'éthylène et d'oxyde d'alkylène comprenant de 3 à 4 atomes de carbone sur un composé comprenant de 3 à 9 groupes hydroxyle, puis réaction avec un oxyde d'éthylène comprenant 4 atomes de carbone.

Lors de la polymérisation d'addition d'oxyde d'éthylène et d'oxyde d'alkylène comprenant de 3 à 4 atomes de carbone sur un composé comprenant de 3 à 9 groupes hydroxyle, les groupes oxyde d'éthylène et oxyde d'alkylène peuvent être polymérisés de manière statistique ou sous forme de blocs.

Parmi les dérivés oxyalkylénés de formule (I), on peut citer notamment le derivé oxyalkyléné (polyoxybutylene polyoxyethylene polyoxypropylene glycerol) representé par la formule (II) suivante :

Gly-[O(PO),ₛ(EO)ₜ-(BO)ᵤH]₃ (I)

dans laquelle :
Gly représente un radical obtenu par élimination de groupes hydroxyle de la glycérine ;
PO représente un groupe oxypropylène ;
EO représente un groupe oxyéthylène ;
s et t représentent respectivement le nombre moyen de moles d'unités PO et EO ont une valeur allant 1 to 50;
le ratio en poids d'unités PO sur EO (PO/EO) va de 1/5 à 5/1;
BO représente un groupe oxyalkylène comprenant 4 atomes de carbone; et
u représente le nombre moyen de moles d'unités BO, et va de 0,5 à 5.

Le derivé oxyalkyléné de formule (II) ci-dessus peut être obtenu par polymérisation d'addition d'oxyde de propylène et d'oxyde d'éthylène sur la glycérine, dans un ratio de 3 à 150 équivalents molaires de chaque oxyde de propylène et oxyde d'éthylène par rapport à la glycérine, suivi de l'addition d'oxyde d'alkylène comprenant 4 atomes de carbone dans un ratio de 1,5 à 15 équivalents molaires par rapport à la glycérine.

La réaction d'addition dudit oxyde d'alkylène sur la glycérine peut se faire en présence d'un catalyseur alcalin, d'un catalyseur de transfert de phase, d'un catalyseur acide de Lewis, ou un équivalent. On utilise en général un catalyseur alcalin, de préférence l'hydroxyde de potassium.

Parmi les dérivés oxyalkylénés de formule (I) ou (II), on préfère les dérivés obtenus par addition de 6 à 10 moles d'oxyde d'éthylène et 3 à 7 moles d'oxyde de propylène sur la glycérine, suivi de l'addition de 2 à 4 moles d'oxyde de butylène. A titre de dérivé oxyalkyléné de formule (I) ou (II) préféré, on peut citer le polyoxybutylene polyoxyethylene polyoxypropylene 8/5/3 glycerol , qui est obtenu par réaction d'addition de 8 moles d'oxyde d'éthylène et 5 moles d'oxyde de propylène sur la glycérine, puis ajout de 3 moles d'oxyde de butylène, et dont le nom INCl est PEG/PPG/polybutylene glycol-8/5/3 glycerin. Le PEG/PPG/polybutylene glycol-8/5/3 glycerin est commercialement disponible sous la référence WILBRIDE S-753 auprès de la société NOF Corporation.

Le ou les dérive(s) oxyalkyléné(s) de formule (I) peuvent être présents dans la composition selon l'invention en une teneur allant de 0,1 à 10 % en poids, de préférence de 0,1 à 7 % en poids et mieux de 0,1 à 3 % en poids par rapport au poids total de la composition.

Avantageusement, la base xanthique et le composé oxyalkyléné de formule (I) décrits précédement sont présents dans la composition selon l'invention dans un rapport pondéral base xantique/composé oxyalkyléné allant de 1 à 50, de préférence allant de 10 à 40, préférentiellement allant de 15 à 25.

La composition selon l'invention comprend un milieu aqueux.

La composition peut comprendre de l'eau en une teneur allant de 30 % à 80 % en poids, par rapport au poids total de la composition, de préférence allant de 40 % à 70 % en poids, et préférentiellement allant de 45 % à 65 % en poids.

L'eau peut être une eau florale telle que l'eau de bleuet et/ou une eau minérale telle que l'eau de VITTEL, l'eau de LUCAS ou l'eau de LA ROCHE POSAY et/ou une eau thermale.

La phase aqueuse peut comprendre au moins un monoalcool ayant de 2 à 6 atomes de carbone tels que l'éthanol, l'isopropanol, notamment en une teneur allant de 1 % à 30 % en poids, par rapport au poids total de la composition, et de préférence allant de 5 % à 20 % en poids.

La phase aqueuse peut comprendre en outre de l'acide salicylique ou l'un de ses dérivés comme l'acide n-octanoyl-5-salicylique, notamment présent en une teneur allant de 0,1 % à 5 % en poids, par rapport au poids total de la composition, de préférence allant de 0,1 % à 3 % en poids, et préférentiellement allant de 0,1 % à 2 % en poids.

La présence de monoalcool et d'acide salicylique dans la phase aqueuse permettent de favoriser une bonne dissolution de la base xanthique dans la phase aqueuse et donc d'améliorer l'efficacité amincissante de la composition.

La composition selon l'invention peut se présenter de préférence sous forme de gel aqueux ou d'émulsion huile-dans-eau.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les agents hydratants (tels que la glycérine, le propylène glycol, le butylène glycol, le pentylène glycol, l'hexylène glycol, le dipropylène glycol, le diéthylène glycol), les conservateurs, les antioxydants, les solvants, les parfums, les charges, les pigments, les filtres hydrophiles, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition selon l'invention est une émulsion, la proportion de la phase grasse peut aller de 5 à 50 % en poids, et de préférence de 5 à 30 % en poids par rapport au poids total de la composition.

Comme matières grasses utilisables dans l'invention, on peut utiliser les huiles minérales, les huiles d'origine animale, les huiles de synthèse, les huiles siliconées et les huiles fluorées. On peut aussi utiliser comme matières grasses des acides gras, des cires et des gommes et en particulier les gommes de silicone.

Les émulsionnants et les co-émulsionnants éventuellement utilisés dans la composition sous forme d'émulsion sont choisis parmi ceux classiquement utilisés dans le domaine considéré. Ces émulsionnants et coémulsionnants sont de préférence présents, dans la composition, en une proportion allant de 0,3 à 20 % en poids, et de préférence de 0,5 à 5 % en poids par rapport au poids total de la composition. Comme émulsionnants et coémulsionnants utilisables dans l'invention, il est particulièrement avantageux d'utiliser les esters d'acide gras et de polyol tels que le stéarate de PEG-100, le stéarate de PEG-50 et le stéarate de PEG-40 ; le tristéarate de sorbitane, les stéarates de sorbitane oxyéthylénés disponibles sous les dénominations commerciales Tween^{®} 20 ou Tween^{®} 60, par exemple ; et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Bien entendu, l'homme de l'art veillera à choisir le ou les éventuels composés à ajouter aux compositions selon l'invention, ainsi que leur concentration, de manière telle que les propriétés avantageuses attachées intrinsèquement aux compositions conformes à l'invention ne soient pas, ou substantiellement pas, altérées par l'addition envisagée.

La composition définie précédemment peut être utilisée pour prévenir ou lutter contre la cellulite et/ou pour affiner la silhouette ou les contours du visage.

L'invention sera maintenant illustrée à l'aide des exemples non limitatifs suivants.

### Exemples 1 et 2 comparatifs :

On a réalisé 1 composition amincissante selon l'invention (exemple 1) contenant un composé polyoxybutylene polyoxyethylene polyoxypropylene glycerol, et une composition hors invention (exemple 2) ne contenant pas ce composé.

On a appliqué la composition sur la peau et observé les propriétés cosmétiques obtenues. L'évaluation a été faite avec un panel de 10 femmes.

Les teneurs sont exprimées en pourcentage pondéral.

| **COMPOSITION** | **Exemple** **1 *** | **Exemple** **2*** |
|---|---|---|
| **PHASE I** | | |
| Eau | qsp | qsp |
| Gélifiant acrylique(1) | 0,4 | 0,4 |
| Gélifiant acrylique (2) | 0,25 | 0,25 |
| Gomme de Xanthane | 0,1 | 0,1 |
| AMPS (3) | 1 | 1 |
| Glycérine | 5 | 5 |
| PEG/PPG/polybutylene glycol-8/5/3 glycerin (4) | 2 | |
| Propylène glycol | 6 | 6 |
| Caféine | 5 | 5 |
| Acide salicylique | 1,7 | 1,7 |
| Triéthanolamine | 2,3 | 2,3 |
| Acide n-octanoyl-5- salicylique | 0,1 | 0,1 |
| | | |

| **PHASE II** | | |
|---|---|---|
| Diméthicone 100 cst (5) | 10 | 10 |
| Parfum | 0,1 | 0,1 |
| | | |

| **PHASE III** | | |
|---|---|---|
| Ethanol | 15 | 15 |
| | | |

| **PHASE IV** | | |
|---|---|---|
| Poudre expansée (6) | 0,1 | 0,1 |
| | | |
| | | |
| Propriétés cosmétiques | Effet doux et glissant, pas collant. | Pas d'effet doux. La peau accroche et tire. Sensation collante. |

| | | |
|---|---|---|
| * : exemple hors invention | | |

On a constaté que la composition de l'exemple 1 selon l'invention, après application sur la peau, présente une bonne douceur et n'est pas collante au toucher tandis que la composition de l'exemple 2 hors invention confère un tiraillement à la peau (pas de douceur) et présente un aspect collant.

### Exemple 3

On a préparé la composition amincissante suivante (teneurs en pourcentage pondéraux) :

| Eau | qsp |
|---|---|
| Gomme de Xanthane | 0,2 |
| AMPS (3) | 2 |
| Glycérine | 5 |
| Propanediol | 5 |
| PEG/PPG/polybutylene glycol-8/5/3 glycerin (4) | 0,25 |
| Caféine | 5 |
| Acide salicylique | 2 |
| Triéthanolamine | 2,3 |
| Acide n-octanoyl-5- salicylique | 0,1 |
| Alcool cétylique | 0,25 |
| Diméthicone 100 cst (5) | 8,5 |
| Polyisobutène hydrogéné (PARLEAM de chez NOF Corporation) | 2 |
| Parfum | 0,1 |
| Mélange de stéarate de glycéryle et de PEG-100 stéarate | 0,15 |
| Ethanol | 5 |
| Poudre expansée (6) | 0,15 |

La composition appliquée sur le corps est douce et non collante.

Ingrédients utilisés :
(1) Polymère carboxyvinylique vendu sous la dénomination « CARBOPOL 980 » par la société NOVEON.
(2) Polymère acide acrylique/acrylate d'alkyles réticulé vendu sous la dénomination « PEMULEN TR-2 » par la société LUBRIZOL
(3) Acide polyacrylamido propane sulfonique neutralisé partiellement à l'ammoniaque et réticulé, vendu sous la dénomination « HOSTACERIN AMPS » par la société CLARIANT.
(4) commercialement disponible sous la référence WILBRIDE S-753 auprès de la société NOF Corporation
(5) Polydimethylsiloxane 100 cst vendu sous la dénomination « BELSIL DM 100 » par la société WACKER
(6) micro-sphères de copolymère chlorure de vnylidene/acrylonitrile/ olyméthylmétanhacrylate expancées à l'isobutane (EXPANCEL 551 DE 40 D42 de chez Expancel)
(7) ARLACEL^{®} 165 FL de chez Uniqema

## Revendications

1. Composition comprenant, dans un milieu aqueux physiologiquement acceptable, au moins une base xanthique ou un extrait végétal en contenant, et au moins un derivé oxyalkyléné de formule (I) suivante:
Z-{O(AO)ₗ(EO)ₘ-(BO)ₙH}ₐ (I)
dans laquelle
Z représente un radical obtenu par élimination d'un ou plusieurs groupes hydroxyle d'un composé comprenant de 3 à 9 groupes hydroxyle ;
AO représente une groupe oxyalkyléné comprenant de 3 à 4 atomes de carbone EO représente un groupe oxyéthylène;
BO représente un groupe oxyalkylène comprenant 4 atomes de carbone, a va de 3 à 9;
I, m, and n représentent respectivement le nombre moyen de moles d'unités AO, EO and BO, et 1 ≤ l ≤ 50, 1 ≤ m ≤ 50 et 0.5 ≤ n ≤ 5;
le ratio en poids de AO sur EO (AO/EO) allant de 1/5 à 5/1.

2. Composition selon la revendication précédente, **caractérisée par le fait que** la base xanthique est choisie parmi : la caféine, la théophylline, la théobromine, l'acéfylline, le nicotinate de xanthinol, la diniprophylline, la diprophylline, l'étamiphylline et ses dérivés, l'étophylline, la proxyphylline, la pentophylline, la propentophylline, la pyridophylline et la bamiphylline.

3. Composition selon la revendication précédente, **caractérisée par le fait que** la base xanthique est la caféine.

4. Composition selon l'une quelconque des revendications précédentes, **caractérisée par le fait que** la base xanthique est présente en une teneur allant de 0,5 % à 10 % en poids, par rapport au poids total de la composition, de préférence allant de 1 % à 7 % en poids, et préférentiellement allant de 2 % à 7 % en poids.

5. Composition selon l'une quelconque des revendications précédentes, **caractérisée par le fait que** le dérivé oxyéthyléné est choisi parmi les composés de formule (II) suivante :
Gy-[O(PO)ₛ(EO)ₜ-(BO)ᵤH]₃ (I)
dans laquelle :
Gly représente un radical obtenu par élimination de groupes hydroxyle de la glycérine ;
PO représente un groupe oxypropylène ;
EO représente un groupe oxyéthylène ;
s et t représentent respectivement le nombre moyen de moles d'unités PO et ont une valeur allant 1 to 50;
le ratio en poids d'unités PO sur EO (PO/EO) va de 1/5 à 5/1;
BO représente un groupe oxyalkylène comprenant 4 atomes de carbone; et
u représente le nombre moyen de moles d'unités BO, et va de 0,5 a 5.

6. Composition selon l'une quelconque des revendications précédentes, **caractérisée par le fait que** le dérivé oxyéthyléné est choisi parmi le polyoxybutylene polyoxyethylene polyoxypropylene 8/5/3 glycerol.

7. Composition selon l'une quelconque des revendications précédentes, **caractérisée par le fait que** le dérivé oxyéthyléné est présent en une teneur allant de 0,1 à 10 % en poids, de préférence de 0,1 à 7 % en poids et mieux de 0,1 à 3 % en poids par rapport au poids total de la composition.

8. Composition selon l'une quelconque des revendications précédentes, **caractérisée par le fait qu'**elle comprend de l'eau en une teneur allant de 30 % à 80 % en poids, par rapport au poids total de la composition, de préférence allant de 40 % à 70 % en poids, et préférentiellement allant de 45 % à 65 % en poids.

9. Composition selon l'une quelconque des revendications précédentes, **caractérisée par le fait qu'**elle comprend un monoalcool ayant de 2 à 6 atomes de carbone.

10. Composition selon la revendication précédente, **caractérisée par le fait que** le monoalcool est choisi parmi l'éthanol, l'isopropanol.

11. Composition selon la revendication 9 ou 10, **caractérisée par le fait que** le monoalcool est présent en une teneur allant de 1 % à 30 % en poids, par rapport au poids total de la composition, et de préférence allant de 5 % à 20 % en poids.

12. Composition selon l'une quelconque des revendications 9 à 11, **caractérisée par le fait qu'**elle comprend de l'acide salicylique ou l'un de ses dérivés.

13. Composition selon la revendication précédente, **caractérisée par le fait que** l'acide salicylique ou ses dérivés est présent en une teneur allant de 0,1 % à 5 % en poids, par rapport au poids total de la composition, de préférence allant de 0,1 % à 3 % en poids, et préférentiellement allant de 0,1 % à 2 % en poids.

14. Composition selon l'une quelconque des revendications précédentes, **caractérisée par le fait qu'**elle se présente sous la forme d'émulsion huile-dans-eau.

15. Procédé cosmétique pour lutter contre la cellulite et/ou la peau d'orange et/ou amincir la silhouette, comprenant l'application topique sur la peau d'une composition selon l'une quelconque des revendications précédentes.
